# EUROPEAN PATENT SPECIFICATION

(11) **EP 3 335 660 B1**
(45) Date of publication and mention of the grant of the patent: **20.01.2021**
(21) Application number: 16204195.8
(22) Date of filing: 14.12.2016
(51) Int. Cl.: A61B 18/24, A61B 18/22, A61B 18/00, A61B 18/18, A61N 5/06

(54) **APPARATUS FOR CONTROLLING LASER THERMOTHERAPY**
VORRICHTUNG ZUR STEUERUNG VON LASERTHERMOTHERAPIE
APPAREIL DE COMMANDE DE THERMOTHÉRAPIE LASER

(43) Date of publication of application: 20.06.2018
(73) Proprietor: Clinical Laserthermia Systems AB, 223 81 Lund (SE)
(72) Inventor: Dymling, Stephan, 216 22 Limhamn (SE); Pantaleone, Cristina, 222 40 Lund (SE)
(74) Representative: KIPA AB

(56) References cited:
- WO-A1-97/07735
- WO-A1-2015/160064
- WO-A2-2012/092508
- US-A- 5 542 916
- US-A- 5 599 345
- US-A1- 2001 056 278
- US-B1- 6 418 337
- US-B1- 6 689 127

## Description

### BACKGROUND OF THE INVENTION

### FIELD OF THE INVENTION

This invention pertains in general to the field of interstitial thermotherapy of a treatment lesion associated with at least an area of tissue, such as a tumour. More particularly the invention relates a system for controlled heating and destruction of cancer using a heat source. Even more particularly the invention further relates to arranging a heat source in the tissue to be treated using a slideable sleeve.

### DESCRIPTION OF THE PRIOR ART

It is known in the art that a tumour may be destroyed by heat, such as thermotherapy. One of the most common thermotherapy techniques is interstitial laser hyperthermia, which destroys tumours by absorption of light. Early experimental and clinical studies used an Nd-YAG laser and bare end fibres inserted into the centre of a tumour. Most of these lacked adequate control of the tissue effect. Methods to improve lesion size included multi-fibre systems, diffuser type fibres and vascular inflow occlusion. However the standard application of interstitial laser hyperthermia results in evaporisation and carbonisation of tissue and relatively unpredictable tissue damage and lesion size.

The treatments are mostly controlled by measuring the temperature either close to the emitting area to avoid over heating close to the heating probe, which means that the control over the size of the treatment lesion is very limited. Another way of controlling the temperature is to insert one or more separate leads having temperature sensor in and/or outside the boundaries of the tumour. This may have both practical and ethical disadvantages. Ethically, more sticks have to be conducted in and around the tumour which may be painful for the patient and increase the risks of track seeding. Practically, it may be hard to arrange the different leads and the temperature sensors at the right positions which may affect the treatment negatively.

Studies from rats and humans have shown that heat treatment of cancer may give rise to an anti-tumour immunologic effect. If the dying tumour cells release uncoagulated tumour antigens, these antigens may produce an immune response when presented to the immune system of the host. Thus, the treated tumour will not only be destroyed but the immune effect will destroy remaining tumour, locally or at distant sites, including lymph nodes. The immunologic effect contributes to the selective tissue damage and the relatively small release of growth factors. The low treatment morbidity gives the possibility to use chemotherapy in a more efficient way since chemotherapy can be started before or at the time of local therapy.

US 6,418,337 describes a MRI guided surgical apparatus includes a heat source formed by a laser and an optical fiber carrying the heat energy into a part to be coagulated by hyperthermia with an end reflector to direct the energy in a beam to one side of the fiber end. The fiber includes a reinforcing sleeve along its length to prevent bending and twisting.

Another apparatus for laser thermotherapy is known from WO 2015/160064 A1.

Until now there has been no real way of fully controlling and/or optimize the treatment lesions in an easy way for the practitioner without the drawbacks of the prior art. This has implications on the possibilities to obtain an optimised treatment for the patients. The current systems and the associated drawbacks also affect the possibilities of obtaining and controlling the immunologic effect. Thus improved control of heat stimulation to optimise the treatment lesion would be advantageous and may increase patient safety. An improved control of the treatment may minimise evaporisation and carbonisation of tissue surrounding the heat source and the adverse effects associated therewith. Further, an improved control over the treatment lesion may improve the possibilities to obtain an immunologic effect. For treatments with the goal of achieving coagulative temperatures the lack of accurate monitoring of the progression of necrotised tissue over time is a limiting factor. To facilitate this, a well-defined temperature measuring technique inside the treatment volume is needed.

### SUMMARY OF THE INVENTION

The invention is defined in claim 1. Further aspects and preferred embodiments are defined in the dependent claims. Aspects, embodiments and examples of the present disclosure which do not fall under the scope of the appended claims do not form part of the claimed invention and are merely provided for illustrative purposes.

Accordingly, examples of the present disclosure preferably seek to mitigate, alleviate or eliminate one or more deficiencies, disadvantages or issues in the art, such as the above-identified, singly or in any combination by providing an apparatus, a system, and a method for controlling a heat treatment of a tumour for providing treatment of a tissue, such a tumour, according to the appended patent claims.

The apparatus, system and method disclosed herein may be used for controlling a process for irreversibly tissue damage, such as a tumour, for treatment purpose. The damage may be obtained by ablation for removing tissue by evaporation or sublimation. Another example is to achieve coagulative temperatures with our without monitoring the progress of necrotised tissue over the time of the treatment, such as focal laser ablation (FLA), sometimes also referred to as laser-induced interstitial thermotherapy (LITT). The treatment may also be improved by obtaining an anti-tumour effect, such as an immunologic effect. The anti-tumour effect may be a local, distant or combined local/distant effect following local tumour destruction. The anti-tumour effect is triggered by antigens and may destroy any part left of a treated tumour but may also destroy other untreated tumours in the patient. Thus the effect may be seen as a "vaccine" against a tumour (abscopal effect). The antigens are a result of a treatment causing cell death but without coagulating/denaturation of tumour antigens.

According to aspects of the disclosure, an apparatus for performing thermotherapy on at least a portion of tissue, such as at least a portion of a tumour, is described. The apparatus comprises a heating probe which comprises an energy emitting area. The heating probe is connectable to an energy source for heating the portion of tissue by the energy emitting area. The apparatus further includes a sleeve and the heating probe is arrangable in the sleeve, and the sleeve is configured to be slid along the heating probe in a distal and/or proximal direction for allowing positioning of the energy emitting area in the portion of tissue for controlling the thermotherapy.

This arrangement has an improved accuracy and makes it easier to positioning the energy emitting area at the right location in the portion of tissue to be treated.

The heating probe may use radiofrequency (RF), microwave frequency (MW), or preferably a laser for heating the tissue by the energy emitting area.

In some examples, the sleeve includes at least one temperature measuring element. By sliding the sleeve with the temperature measuring elements in a distal and/or proximal direction, the temperature measuring elements may be positioned at an optimal distance in relation to the energy emitting area. This arrangement has an improved accuracy for controlling the thermotherapy and the size of the treatment lesion compared to using a temperature sensor positioned outside the portion of tissue to be treated by a separately inserted lead. One reason is that the temperature sensor will be aligned with the emitting area which can be very hard to achieve when positioning a sensor outside the tumour using a separate lead. When positioning a temperature sensor outside the tumour using a separate lead, the temperature sensor will most likely not end up aligned with the emitting area of the heating probe but instead be positioned either too deep, shallow or at a distance too far away. Another advantage is that it is not needed to re-insert the lead with the temperature sensor if the distance between the heating probe and the temperature sensor of the first insertion was considered not good to achieve an optimal treatment lesion.

A further advantage of having the temperature measuring elements arranged in the sleeve, which can be made in a plastic material, is that the temperature measuring points are isolated and will not be affected by a separate lead, which is often made of metal, used for positioning the temperature sensor. By having them isolated the temperature measuring will be faster and/or more accurate as, for example, the temperature measuring elements will not be cooled by the lead as in the case of using a separate lead for positioning the temperature sensor.

In some examples of the disclosure is the at least one temperature measuring element arranged in a channel of the sleeve. The sleeve may thereafter be heated and will shrink around the at least one temperature measuring element. Alternatively, in some examples, the sleeve may comprise two shrink tubing concentrically arranged. The temperature measuring elements 20 may be arranged between the two tubes. The tubes may thereafter be heat shrunk. Alternatively, in some examples at least one temperature measuring element braided or woven into the sleeve.

In some examples of the disclosure, the heat probe comprises a fibre and the light emitting area of the fibre is at least partially a diffuser. For example, in some examples the diffuser is a radial fibre. In some other examples the diffuser is a structured writing in a core and/or cladding and/or buffer of the fibre.

By using a diffuser the radiation profile can be varied compared to using a bare end fibre without a diffuser. For example, if the diffuser is a radial fibre more than one discrete radial radiation point may be used. Each of the radiation point may have the same effect or the effect could differ between the radiation points to obtain a specific radiation profile. Another aim may be to obtain a suitable power density. The radiation profile and/or the power density will affect the treatment and/or the shape of the treatment lesion. The same applies when using a diffuser which is made from a structured writing in a core and/or cladding and/or buffer of the fibre. By changing the pattern, the position, and/or the density of the writing, different radiation profile or power density may be obtained which may be used to optimise the treatment of the portion of tissue, such as a portion of a tumour.

In some examples of the disclosure the sleeve is an introducer catheter.

In some examples of the disclosure are at least two temperature measuring elements arranged at the same transverse plane of the sleeve. This may be done for redundancy to be able to measure the temperature using at least two temperature measuring elements arranged at the same distance from the light emitting area. Should one of the at least two temperature measuring elements give a false value or no value, the other temperature measuring elements may be used instead of the one being broken or damaged. This will improve patient safety and decrease the risk of having to re-insert a new sleeve and heating probe.

In some examples of the disclosure is the emitting area of the heating probe covered by a capillary. The capillary may improve the thermostable properties of the heating probe and keep the emitting area intact at when the surface of the capillary is subjected to high temperatures. The capillary may be bonded and sealed to the fibre or the heating probe by fusing, and/or adhering, using for example glue, and/or shrink tubing. When the heat probe includes a fibre, an advantage of fusing the capillary being a glass capillary to a bare end of the fibre is that a very thermostable bond between silica against silica is obtained at the distal end which is exposed to high temperature. Thereby further improving the thermostable properties of the heating probe and to keep the emitting area intact at high temperatures.

In some examples of the disclosure a hub is used to lock the sleeve and the heating probe when right position, for example when an optimal distance between the at least on temperature element relation to the energy emitting area is found. By having one part of the hub located at the proximal end of the sleeve and a second part of the hub located at the heating probe the two can be fastened together before sliding the sleeve along the heating probe to find the optimal location for the temperature measuring elements. When the right position has been located a locking member, preferably a valve, such as a haemostatic valve, which is part of the hub, may be used to lock the position of the heating probe, such as the fibre, and the sleeve before starting the treatment.

The temperature sensor in the sleeve may be combined with external temperature measuring points, e.g. when sensitive anatomical structures need to be protected. This can serve as a guard shutting off the heat source at a predetermined level to avoid damage to the sensitive structure.

In a further aspect of the disclosure, a system for performing thermotherapy on at least a portion of tissue, such as at least a portion of a tumour, is disclosed. The system includes a heating probe which comprises an energy emitting area connectable to an energy source for heating the portion of said tissue by the energy emitting area. The system also includes a sleeve. The system may also include means for measuring a temperature in said portion of tissue and a display unit for indicating a measured temperature from the means for measuring a temperature. The heating probe is arrangeble in the sleeve the sleeve is configured to be slid along the heating probe in a distal and/or proximal direction for positioning the energy emitting area in the portion of tissue to be treated for controlling the thermotherapy.

The temperature displayed on the display unit may be used for controlling the energy to the energy emitting area and thereby controlling the thermotherapy.

If the means for measuring a temperature are temperature measuring elements arranged in the sleeve, the displayed temperature may be used to find the optimal distance between the energy emitting area and the temperature measuring elements.

When the goal of the treatment is to coagulate tissue the temperature sensor in the sleeve is spatially very well defined and the distance to the heat source is very accurate. Using well known bioheat algorithms tissue damage over time may therefore be monitored.

In some examples, another way of achieving an improved accuracy when measuring the temperature during a treatment is disclosed. This includes using Magnetic Resonance Imaging to obtain a 2D or 3D temperature map of the treatment area. A region of interest (ROI) may be defined by determining a point at a distance, such as at the tumour boarder, from the emitting area. This region will define a treatment lesion and the temperature in this ROI may be used to control the heat source in order to maintain the temperature at a predetermined value. Other regions in the 2D temperature map of the MR Image may also be defined to serve as an automatic safety function shutting down the heat source if a threshold temperature is reached.

In some examples of the disclosure, the target temperature is between 40 to 60°C, such as, 40 to 55°C., such as 42 to 50°C. The temperature may, for example, be indicated as a graph on the display.

Controlling the heating and the treatment of the tumour by monitoring the temperature at the edge of the treatment lesion and to keep the monitored temperature stable in the identified range has shown to give a god treatment of the treatment with improvements related to safety and limited adverse effects for the patient, such as minimise evaporisation and carbonisation of tissue surrounding the heat source and the adverse effects associated therewith. This relates for example to focal laser ablation (FLA) where it is utilized that the extension of thermal tissue damage depends on both temperature and heating duration. Cell viability is in relation with thermostability of several critical proteins. Irreversible protein denaturation may occur around 60°C. While over 60°C, coagulation is quasi-instantaneous, between 42 and 60°C, a thermal damage is obtained with longer heating periods. The area submitted to supraphysiological hyperthermia less than 60°C will develop coagulative necrosis in 24 to 72 h after treatment.

Additionally, by keeping the temperature at the edge of the treatment lesion within the range 42 to 50°C, such as 44 to 48°C, range has been shown to have the prospect of providing an anti-tumour immunologic effect against the treated cancer, i.e. immunostimulating laser thermotherapy.

It should be emphasized that the term "comprises/comprising" when used in this specification is taken to specify the presence of stated features, integers, steps or components but does not preclude the presence or addition of one or more other features, integers, steps, components or groups thereof.

### BRIEF DESCRIPTION OF THE DRAWINGS

These and other aspects, features and advantages of which examples of the disclosure are capable of will be apparent and elucidated from the following description of examples of the present invention, reference being made to the accompanying drawings, in which
Fig. 1 is a schematic illustration over an exemplary apparatus for controlling a heat treatment of tissue, such as a tumour;
Fig. 2 is a schematic illustration of an exemplary setup of an apparatus for heat treatment of tissue;
Fig. 3A to D are a schematic illustration of an exemplary introduction system and heat probe;
Fig. 4A to 4B are a schematic illustration of positioning of a temperature sensor integrated into a sleeve, such as an introducer;
Fig. 5A to D are a schematic illustration of an introducing system with a hub.
Fig, 6 is a schematic illustration of a probe and introducer arranged in a tumour for obtaining a lesion;
Fig. 7A and B are a schematic illustrations of a capillary arranged around a diffusing fibre distal end; and
Fig.8 is a schematic illustration of a method for heat treatment of tissue, such as a tumour.

### DESCRIPTION OF THE PREFERRED EXAMPLES

Specific examples of the discloser will be described with reference to the accompanying drawings. This disclosure may, however, be embodied in many different forms and should not be construed as limited to the examples set forth herein; rather, these examples are provided so that this disclosure will be thorough and complete, and will fully convey the scope of the disclosure to those skilled in the art. The terminology used in the detailed description of the examples illustrated in the accompanying drawings is not intended to be limiting of the disclosure. In the drawings, like numbers refer to like elements.

The following description focuses on examples applicable to a device, system, and method for controlling thermotherapy of tissue. The thermotherapy is controlled by controlling a size of a treatment lesion covering at least a portion of tissue to be treated, such as a portion of a tumour. Preferably the treatment lesion is sized to the whole tissue area to be treated, such as a tumour, by positioning the temperature measuring element used for controlling the treatment outside the tissue area. Preferably the temperature measuring element used for controlling the treatment is positioned about 2 to 5 mm outside the boundary of the tissue to be treated, such as a portion of a tumour. In particular the disclosure relates to a device, system, and method for obtaining coagulative temperatures and accurately monitoring the progression of necrotised tissue over time or obtaining an anti-tumour immunologic response by thermotherapy of at least a portion of a tumour. However, it will be appreciated that the invention is not limited to this application but may be applied to other areas of thermotherapy treatment of tumours.

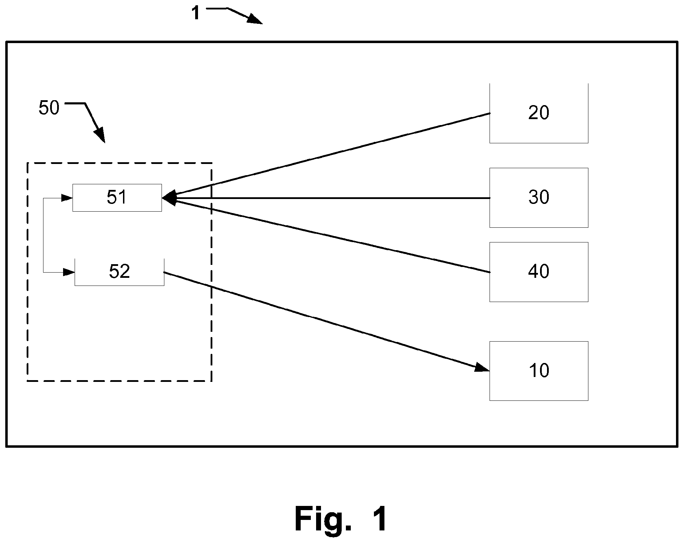

In an example according to Fig. 1, a schematic illustration over an exemplary system 1 for thermotherapy of tissue, such as a tumour, is illustrated. The system 1 is especially developed for controlling the thermotherapy by controlling the size of a lesion covering at least a portion of a tumour. The system 1 may in some examples be used for controlling immunostimulating laser thermotherapy.

The system 1 comprises a control unit 50 which includes a temperature reading unit 51 configures to receive temperature data from a temperature measuring element 20. The temperature measuring elements may be a temperature sensor, such as a thermistor/thermocouple. In some examples of the system, the temperature measuring element 20 is arranged in a sleeve. The temperature measuring element 20, such as thermistor/thermocouples, may be arranged in a channel of the sleeve, such as a multi-lumen sleeve. The sleeve may be heat shrunk after the temperature measuring elements have been arranged in the channels of the sleeve. Alternatively, in some examples, the sleeve may comprise two shrink tubing concentrically arranged. The temperature measuring elements 20 may be arranged between the two tubes. The tubes may thereafter be heat shrunk. Another alternatively, in some example, the temperature measuring element 20 may be braided or woven into the sleeve.

Alternatively and/or additionally to have the temperature sensors arranged in the sleeve, Magnetic Resonance Imaging may be used to obtain a 2D or 3D temperature maps of the treatment area. A region of interest (ROI) may be defined by determining a point at a distance, such as at the tumour boarder, from the emitting area. This region will define a treatment lesion and temperature in this ROI may be used to control the heat source in order to maintain the temperature at a predetermined value. Other regions in the 2D temperature map of the MR Image may also be defined to serve as an automatic safety function shutting down the heat source if a threshold temperature is reached.

When using an external temperature measuring sensor, such as a Magnetic Resonance Imaging system, instead of temperature measuring elements arranged in the sleeve, the sleeve probe arrangement is used for improving and making it easier to positioning the heating area in the tissue.

A heating probe 10 having an energy emitting area is arrangable in the sleeve. The heating probe 10 may comprise a fibre having a light emitting area at the distal end, wherein the distal end is configured to be interstitially arranged in the tumour. Alternatives to a fibre may be to use radio frequency RF) or Microwaves (MW) to heat the tissue by the energy emitting area. In some examples, the heating probe 10 has capillary arranged over the energy emitting area, such as the light emitting area. Wherein the capped end of the fibre is the distal end, and wherein the distal end is configured to be interstitially arranged in the tissue, such as the tumour. The capillary may, especially when a fibre is used as a heat probe, improve the heat and mechanical stability of the heat probe. In a further example, the light probe is only a fibre having a light emitting area at the distal end, wherein the distal end is configured to be interstitially arranged in the tumour.

When in use, the sleeve may be movably slid along the heating probe 10 in a distal and/or proximal direction to allow positioning of the temperature measuring element 20 at the right distance in relation to an energy emitting area of the heating probe 10 for controlling the thermotherapy by controlling the size of the lesion. The sleeve may be a sleeve arranged around the heating probe 10. The heating probe 10 and the sleeve may be positioned inside an introduced when performing the thermotherapy. Alternatively, the sleeve may be the introduced and the heating probe 10 is arranged inside the introducer when performing the thermotherapy.

Additionally, in some examples of the system the sleeve may have a plurality of temperature measuring elements, 20, 30, 40 arranged in the sleeve. The plurality of temperature measuring elements, 20, 30, 40 may be arranged spaced apart along the sleeve. When using a plurality of temperature measuring elements, 20, 30, 40, the number of temperature measuring elements may be any number larger than 1, such as 2 to 20, such as 2 to 15, such as 2 to 10, such as 2 to 5.

Alternatively, in some examples, at least temperature measuring elements, 20, 30, 40 may be arranged at the same position instead of being spaced apart. This may be done for redundancy to be able to measure the temperature using at least two temperature measuring elements, 20, 30, 40 arranged at the same distance from the emitting area, such as a light emitting area. Should one of the temperature measuring elements, 20, 30, 40 give a false value or no value, the other temperature measuring elements, 20, 30, 40 may be used instead of the one being broken or damaged.

Alternatively, in some examples the temperature measuring elements 20, 30, 40 may be arranged in the heating probe 10 instead of the sleeve. In some examples, temperature measuring elements, 20, 30, 40 may be arranged in both the sleeve and the heating probe 10. Additionally, in some examples, the system 1 may include further external temperature measuring points. These external temperature measuring points may be positioned, for example, next to sensitive anatomical structures that need to be protected from high temperature. These external temperature measuring points may therefore serve as guards shutting off the heat source at a predetermined level to avoid damage to the sensitive structure.

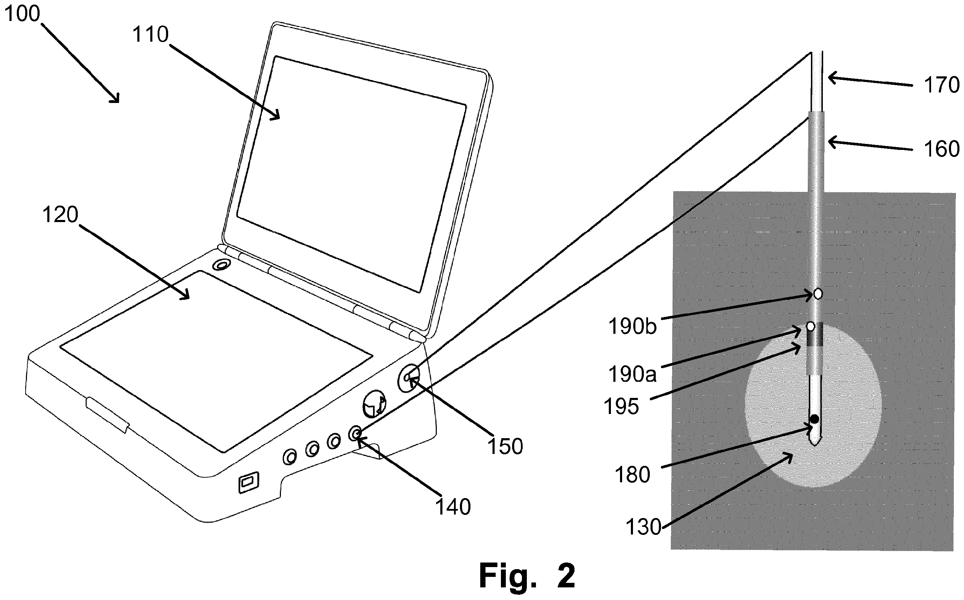

The control unit 50 further comprises a power controlling unit 52, such as an energy source, for controlling the energy emitted by the heat probe 10. The energy causing the heating of the tissue may be emitted using for example RF technology or laser technology. Laser technology may be preferred as it has been shown to improve the control and heating of the tissue to be treated thereby improving the accuracy of optimizing the treatment lesion. Fig. 2 illustrates a schematic drawing of a system for thermotherapy of tissue, such as a tumour. The system comprises a control unit 100. The control unit 100 may for example be a computer connected to power control unit to be connected to a heating probe 170. The heating probe could be an RF probe, a MW probe, or preferably an optical fibre being connectable to a laser unit. The control unit may also include a temperature reading unit to be connected to temperature measuring elements 190a, 190b. The control unit 100 may further include a display unit 110 for displaying information to a practitioner. The information could be, for example, current measured temperature; time lapsed of the treatment; graphs showing changes in the measured temperature over time; size of the lesion; and the power to the heating probe 170. The control unit further includes an input unit 120, such as a keyboard, a computer mouse, a touch pad, or a touch screen. The control unit 100 may further have a first port 150 for connecting the heating probe 170 to the control unit 100. The control unit 100 may also have a second port 140 for connecting at least one temperature measuring element 190a, 190b, to the control unit 100.

In the system illustrated in Fig. 2, a heating probe 170 is connected to a control unit 100 via the port 150. The heating probe may be an RF probe, MW probe, or preferably is an optical fibre. The heating probe 170 is connectable to an energy source (not shown), such as a laser source, RF source or MW source, controlled by a power control unit (not shown), such as a laser driver, of the control unit 100.The heating probe 170 has an emitting area at the tip 180. The emitting area may be a light emitting area. The emitting area is configured to be arranged interstitially in the tissue, such as a tumour, to be treated using the sleeve 160, such as an introducer. When applying energy for heating the tissue, a treatment lesion 130 is obtained covering at least a portion of the tissue to be treated.

In the illustrated examples of the heating probe 170 being an optical fibre, and the emitting area is a light emitting area. The light emitting area may be a bare fibre end or a diffuser. In some examples, the diffuser is a radial fibre. In some other examples, the diffuser is a structured writing in a core and/or cladding and/or buffer of the optical fibre.

Additionally, in some examples the emitting area may be covered with a capillary to improve the heat and mechanical stability during the treatment.

In the schematic illustration, the heating probe 170 is movably arranged as it may slide in an introducer 160. In the introducer is at least one temperature measuring element, 190a, 190b arranged in accordance with the examples given for Fig. 1. In the schematic illustrated figure two temperature measuring element, 190a, 190b are arranged spaced apart along the sleeve. Additionally and/or alternatively, in some examples of the introducer 160, at least two temperature measuring elements, are arranged at least position 190a so that the at least two temperature measuring elements are arranged in the same transverse plane of the sleeve. This may be done for redundancy to be able to measure the temperature using at least two temperature measuring elements arranged at the same distance from the emitting area, such as a light emitting area. Should one of the temperature measuring elements arranged in the same transverse plane of the sleeve give a false value or no value, the other temperature measuring elements may be used instead of the one being broken or damaged.

After the introducer 160 and the heating probe 170 have been arranged in the tumour, the size of the treating lesion may be determined by sliding the introducer to increase or decrease the distance between the emitting area and the at least one temperature measuring element 190a, 190b. The at least one temperature measuring elements 190a, 190b is used for measuring the temperature which is used to control the heating of the tissue by increasing or decreasing the power to the heating probe, such as adjusting the power to the laser unit connected to the control unit 100.

Additionally and/or alternatively, in some examples when at least two measuring points 190a, 190b are used, if a first measuring point 190a measures a to high temperature after adjustment by sliding the introducer 160, a second measuring point 190b having a longer distance to the emitting area may be used for controlling the treatment.

Additionally and/or alternatively, in some examples when at least two measuring points 190a, 190b are used, if a first measuring point 190a measures and a to high temperature and a second measuring point 190b measures a to low temperature after adjustment by sliding the introducer 160 a virtual point located between the first and the second measurements point 190a, 190b may be used for controlling the treatment. The temperature of the virtual point may be calculated using the measured temperature at the first measuring point 190a and at the second measuring point 190b.

The introducer 160 may in some examples have markers 195 arranged along its length to make it easier to positioning the introducer 160 at the right location by make it visible using ultrasound, MRI, x-ray or other imaging equipment.

As previously described in relation to Fig 1, the system illustrated in Fig. 2 may be combined with a Magnetic Resonance Imaging system which may be used for obtaining a 2D or 3D temperature maps of the treatment area which may be used from defining a treatment lesion. If the system is combined with a Magnetic Resonance Imaging system, the sleeve may not include temperature measuring elements as the temperature at the boarder of the treatment lesion 130 may be measured by the Magnetic Resonance Imaging system.

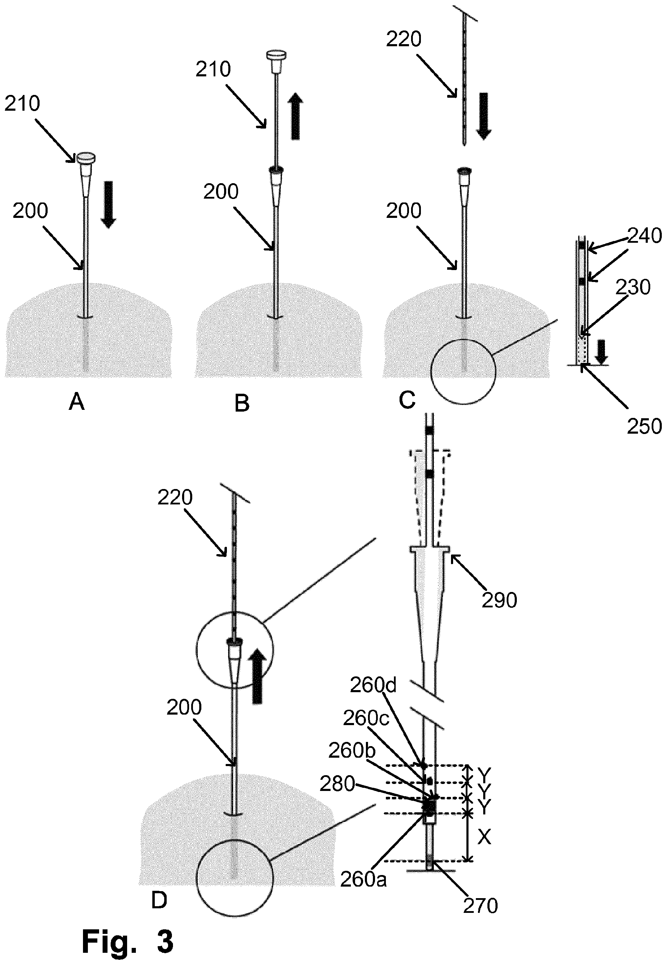

Figure 3A to D illustrate a schematic example of how to positioning the sleeve 200, such as an introducer, and the heating probe 220 in a tumour.

Illustrated in Fig. 3A is an introducer 200 and an introducer stylet 210 used for positioning the introduced in the tissue, such as in the tumour. In Fig. 3B the introducer stylet 210 is removed from the introducer 200. In Fig. 3C the heating probe 220 is arranged in the introducer 200. In this example, the heating probe 220 is pushed until the tip 230 of the heating probe 200 reached the end of the introducer 200. The heating probe 220 may have markers 240 arranged along its length to make it easier to positioning the heating probe 220 at the right location by make it visible using ultrasound, MRI, x-ray or other imaging equipment. After the tip 230 of the heating probe 220 has reached the end 250 of the introducer 200 the introducer may be movably slid along the heating probe 220, as illustrated in in Fig. 3D. By sliding the introducer 200 up and down along the heating probe 220 a distance X will be obtained between the first temperature measuring point 260a and the emitting area 270. Sometimes, the first temperature measuring point 260a may not be used, instead the distance X may be determined between the emitting area 270 and a different temperature measuring point, for example any of temperature measuring points 260b to d.

By monitoring the temperature while sliding the introducer 200 up and/or down along the heating probe 220, the optimal lesion size may be determined. The introduced may have at least one marker 280 for monitoring its position using an imaging modality device, such as ultrasound, x-ray or MRI. The introducer may have further temperature measuring elements 260b to d spaced with a distance Y along the length of the introducer 200.

When the right distance X between the emitting area 270 and the temperature measuring element 260a, or 260b to d, is found, the introducer is locked at its position by a hub 290 which includes a locking member, such as a valve, such as a haemostatic valve. As previously described, in some examples when at least two measuring points 260a, 260b to d are used, if a first measuring point 260a measures a to high temperature by sliding the introducer 200, a second measuring point 260b to d having a longer distance to the emitting area 270 may be used for controlling the treatment and the size of the treatment lesion.

In some examples, a pre-determined distance X is set before positioning the heating probe 220 in the introducer 200. When the emitting area 270 of the heating probe 220 reached the distal end of the introducer 200 the introducer 200 is pulled back until the introducer 200 and the heating probe 220 is locked together at the hub 290. In some further examples, the locking includes sematic feedback to indicate to the practitioner that the introducer 200 has been pulled back to the right position. If a further adjustment of the distance is needed the, locking member, such as a valve, of the hub 290 may be opened and the introducer 200 may be further slid in a distal and/or proximal direction until the right position has been found. Thereafter may the locking member, such as a valve, be closed and the introduced 200 and the heating probe 220 may be locked together before the treatment is started.

In some examples when at least two measuring points 260a, 260b to d are arranged in the sleeve, if a first measuring point 260a measures a too high temperature and a second measuring point 260b to d measures a to low temperature after adjustment by sliding the introducer 200 a virtual point located between the first and the second measurements point 260a, 260b to d may be used for controlling the treatment. The temperature of the virtual point may be calculated using the measured temperature at the first measuring point 260a and at the second measuring point 260b to d.

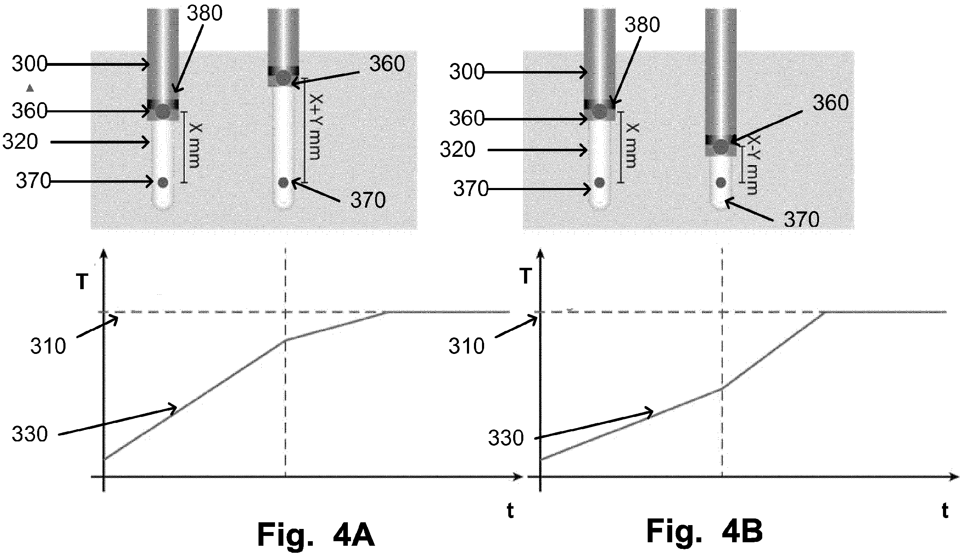

Fig. 4A and 4B are schematically illustrating examples of how to optimize the treatment lesion by sliding a sleeve, such as an introducer, 300 up and/or down along an inserted heating probe 320.

The aim is to optimize the lesion size and to reach the treatment temperature 310, also called the target temperature. The treatment temperature 310 may be in the range 40 to 55°C, such as 44 to 48°C, such as 46°C at the edge of the treatment lesion. Controlling the heating of the tumour by monitoring the temperature at the edge of the treatment lesion and to keep the monitored temperature stable in the ranges identified has shown to give a good outcome of the treatment with improvements related to safety and limited adverse effects for the patient, such as minimise evaporisation and carbonisation of tissue surrounding the heat source and the adverse effects associated therewith. Additionally, keeping the temperature at the edge of the treatment lesion within these ranges has been shown the prospect of providing an anti-tumour immunologic effect against the treated cancer, i.e. immunostimulating laser thermotherapy.

In Fig. 4A, the initial distance between the emitting area 370 of the heating probe 320 and a temperature measuring element 360 arranged in a sleeve, such as an introducer, 300 is X mm. The sleeve 300, may also have a marker 380 for visualizing the position of the distal end of the sleeve 300. If the temperature monitored with the temperature measuring element 360 is determined to increase too fast by observing the temperature curve 330 on a display, the sleeve 300 may be slid so that the distance between the emitting area 370 and the temperature measuring element 360 increases to a distance of X+Y mm. The treatment lesion may in this example be made larger than initially planned for, therefore providing an optimized treatment and keep the target temperature 310 stable.

In Fig. 4B, the initial distance between the emitting are 370 of the heating probe 320 and a temperature measuring element 360 arranged in a sleeve, such as an introducer, 300 is X mm. The sleeve 300, may also have a marker 380 for visualize the position of the distal end of the sleeve 300. If the temperature monitored with the temperature measuring element 360 is determined, by watching the temperature curve 330 on a display, to increase too slowly or the target temperature 310 may not be reached, the sleeve 300 may be slid so that the distance between the emitting area 370 and the temperature measuring element 360 decreases to a distance of X-Y mm. Hence the treatment lesion may be made smaller to be able to optimize the treatment and to keep the target temperature 310 stable.

In figure 4A and 4B the illustration shows that the optimization is done with respect to the most distal temperature measuring element. As previously described, sometimes it may be more practical to use another temperature measuring element or to use a virtual point located between two temperature measuring elements.

Another reason for using a temperature measuring element for optimizing the treatment lesion and for controlling the treatment than the most distal temperature measuring element is that the more distal one may be positioned inside the treatment lesion. The one or more temperature measuring elements positioned inside the treatment lesion, such as inside the tumour, may be used for measuring and controlling other parameters. For example, the temperature measuring element positioned inside the treatment lesion may be used for detecting bleeding and/or carbonization and/or coagulation.

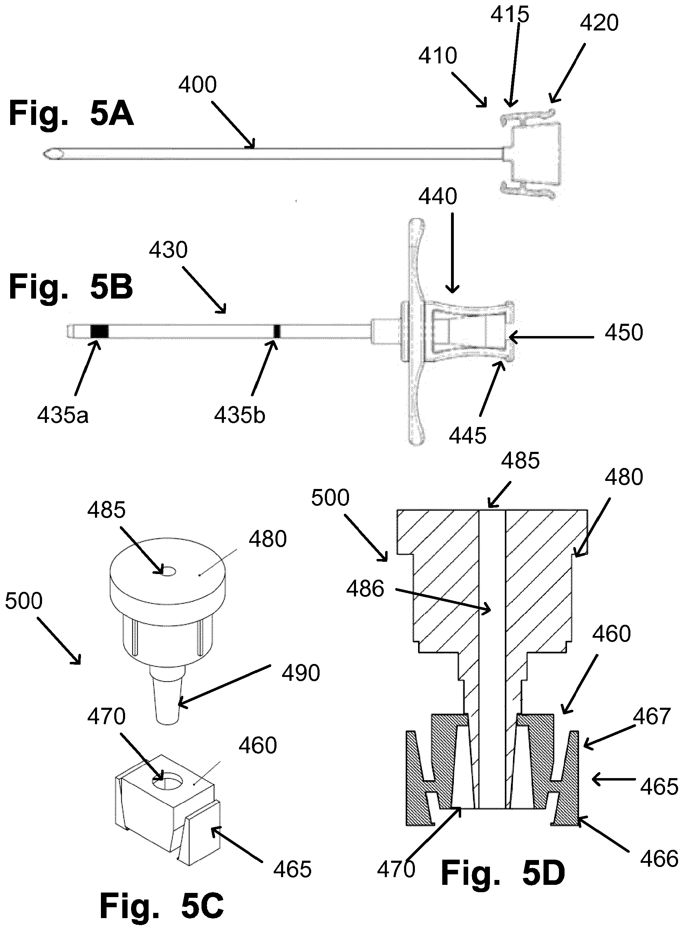

Fig. 5A to 5D are illustrating a sleeve system having a hub, the sleeve system may also be an introducing catheter system. The sleeve system with the hub illustrated in Fig. 5A to 5D may be used with any of the arrangements described and illustrated in relation to Fig. 1 to 4.

Fig. 5A is illustrating an introducer stylet 400 having a connector 410 comprising at its proximal end means for fastening 410 the connector to a hub and means for releasing 420 the connector from a hub. Fig. 5B is illustrating a sleeve 430, such as an introducing catheter. The illustrated sleeve 430 may have markings 435a, 435b for visualizing the positioning of the sleeve in tissue using an imaging modality device, such as ultrasound, x-ray or MRI, but other modalities may also be used. In the sleeve 430, temperature measuring elements (not illustrated), such as thermistor/thermocouples, may be arranged. The temperature measuring elements may be arranged in channels of the sleeve. The sleeve 430 may be heat shrunk after the temperature measuring elements have been arranged in the channels of the sleeve. In other examples, the sleeve 430 may comprise two shrink tubing concentrically arranged. The temperature measuring elements may be arranged between the two tubes. The tubes may thereafter be heat shrunk. Alternatively, in some example, the temperature measuring elements may be braided or woven into sleeve.

The sleeve has a hub 440 attached to its proximal end. The hub 440 has an opening 450 for allowing a stylet as illustrated in Fig 5A or a heating probe to be introduced into the sleeve 430. The hub may also comprise a protruding element 445 for the fastening means, such as the fastening means 415 of the connector 410 at the proximal end of the stylet 400, to hook to and thereby locking the connector to the hub 440. By pressing the releasing means of a connector, such as the releasing means 420 at the connector 410 at the proximal end of the stylet 400, the connecter can be removed from the hub 440.

Fig. 5C is illustrating a second hub 500 which comprises of two parts, a connector 460 and a locking member 480, such as a valve, such as a haemostatic valve. The connector 460 is similar to connector 410 of the stylet and comprises fastening and releasing means 465 for connecting the second hub 500 to the hub 440 at the distal end of the sleeve. The locking member 480 comprises an opening 485 at the proximal end for inserting a heating probe (not illustrated). In Fig. 5C, the second hub 500 is illustrated as comprising two parts wherein, the distal end has a protruding member 490 to be inserted into an opening 470 of the connector 460. These two parts may be assembled and delivered as one unit. Alternatively, the hub 500 may be molded as a single unit instead of being two parts that will be combined.

Fig. 5D is illustrating a cross-section of the second hub 500 after the connector 460 and the locking member 480 has been combined. Fig. 5A is illustrating that the fastening and releasing means 465 includes means for fastening 466 the second hub 500 to the hub 440 of the sleeve by hooking into the protruding element 445. Fig 5D also illustrates that the fastening and releasing means 465 includes means for releasing 467 the second hub 500 from the hub 440 of the sleeve by pressing the means for releasing 467. This type of fastening and releasing means used for the second hub 500 and the stylet 400 allows for a simple and safe way of fastening and releasing the stylet 400 or second hub 500.

In Fig. 5D a lumen 486 is illustrated to go through the locking member 480, such as a valve, and thereby through the second hub 500. The heating probe may be arranged in this lumen 485 during the treatment. By locking the locking member 480, such as closing a valve or closing a haemostatic valve, the heating probe will be locked into its position in the lumen 486. After the heating probe has been arranged in the lumen 486, the heating probe may be arranged in the sleeve 480 after the sleeve has been interstitially inserted in to the tissue, such as a tumour, to be treated. The connector 460 will be fastened to the hub 440, wherein the locking member 480 may be opened and the sleeve 430 may be slid up and/or down along the heating probe. When the right position of the distance between an emitting area of the heating probe and a temperature measuring element of the sleeve 430 has been found, the locking member 480 may be closed to lock the heating probe and the sleeve 430 into the position, whereinafter the heat treatment may start.

The connectors and the hub may be made of a suitable material, for example metal or plastic, such as acrylic.

In some examples, a pre-determined distance is set before arranging the heating probe in the sleeve 430 by positioning the second hub 500 at the pre-determined position on the heating probe. When arranging the heating probe in the sleeve 430 and the emitting area of the heating probe has reached the distal end of the sleeve 430, the sleeve 430 may be pulled back until the hub 440 of the sleeve 430 and second hub 500 of the heating probe is locked together. In some further examples, the locking includes sematic feedback to indicate to the practitioner that the sleeve 430 has been pulled back to the right position and that the hub 440 and the second hub 500 have been locked together. If further adjustments of the distance between the emitting area and the temperature measuring element are needed, the locking member 480, of the second hub 500 may be opened and the sleeve 430 may be further movably slid in a distal and/or proximal direction until the optimal position is found. Thereafter may the locking member 480 be closed and the sleeve 430 and the heating probe may be locked together before the treatment is started.

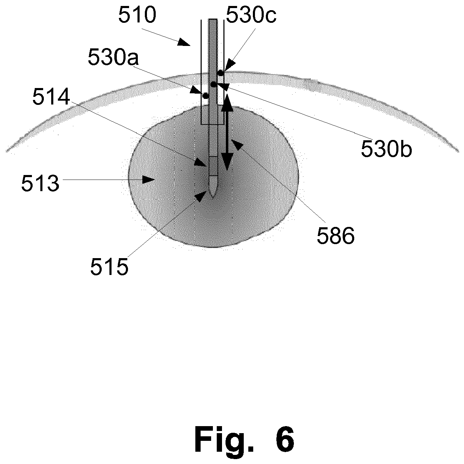

Fig. 6 is illustrating a sleeve 510 and a heating probe 515 arranged inserted into tissue. The heating probe comprises an emitting area 514 and the sleeve comprises at least one temperature measuring element, 530a to c arranged in the sleeve 510. The arrow 586 illustrates that the sleeve 510 may be slid along the heating probe to obtain a distance between the emitting area 514 of the heating probe 515 and the at least one temperature measuring element 530a to c thereby obtaining an optimized size of the treatment lesion 513. The size of the treatment lesion will have a radius which is about the distance between the centre of the emitting area 514 of the heating probe 515 and the at least one temperature measuring element 530a to c of the sleeve 510 used for monitoring the temperature for controlling the energy delivered by the energy source, such as a laser unit, for heating the tissue. As previously described, the treatment temperature at the point for monitoring the temperature, also called the target temperature may be in the range 40 to 60°C, such as 42 to 55, such as 44 to 48°C, such as 46°C. This temperature is monitored at the edge of the treatment lesion. Controlling the heating of the tumour by monitoring the temperature at the edge of the treatment lesion and to keep the monitored temperature stable in the ranges identified has shown to give a good outcome of the treatment with improvements related safety and limited adverse effects for the patients, such as minimise evaporisation and carbonisation of tissue surrounding the heat source and the adverse effects associated therewith. This relates for example to focal laser ablation (FLA) where it is utilized that the extension of thermal tissue damage depends on both temperature and heating duration. For FLA a temperature between 42 and 60°C is normally used and a thermal damage is obtained with longer heating periods compared to normal laser ablation.

Additionally, keeping the temperature at the edge of the treatment lesion within these ranges has been shown prospect for providing an anti-tumour immunologic effect against the treated cancer, i.e. immunostimulating laser thermotherapy. The treatment may preferably be performed for about 30 min after the temperature measuring elements have been positioned at the right distance with respect to the emitting area. Sometimes longer or shorter times may be used..

While the sleeve and heating probe are removed, the emitting area may continue to deliver energy to the surrounding tissue, thereby heating the channel which may minimise the risk of track seeding.

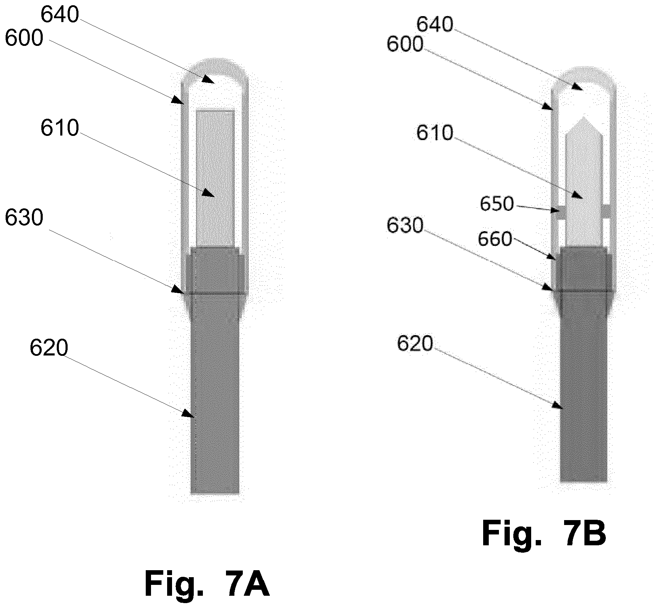

Fig. 7A and 7B are illustrating a capillary to be used to protect the emitting area of, in this example, an optical fibre. The emitting area of the optical fibre may either be a bare end fibre or a diffuser. The diffuser may be any type of diffuser but is preferably either a radial fibre or a structured writing in a core and/or cladding and/or buffer of the optical fibre.

The optical fibre may be, for example, made of silica or a plastic, or may be any other suitable type of optical fibre. The optical fibre may also be a polymer coated fibre.

Fig 7A illustrates a glass capillary 600 covering a bare end fibre 610 which may include a diffuser. The bare end fibre 610 comprises of a fibre core and may also in some examples include a cladding. The tip of the distal end of the fibre 610 may be in some examples flat as illustrated in Fig. 7A or conical. The glass capillary 600 may be bonded 630 to the jacket 620 and/or buffer of the fibre either by fusing or by using an adhesive, and/or by shrink tubing. The space 640 around the bare end fibre 610 may be filled with air. This design of the capillary increases the fibre's mechanical stability and its resistance to high temperatures.

Fig 7B illustrates a similar cap as the one in Fig. 7A. The cap is made from a glass capillary 600 covering a bare end fibre 610 which may include a diffuser. The bare end fibre 610 comprises of a fibre core and may also in some examples include a cladding. The tip of the distal end of the fibre 610 may be in some examples flat or conical as illustrated in Fig. 7B..

The glass capillary 600 and the fibre 610 are fused together at the point 650 which means that a very thermostable bond between silica and silica is obtained at the distal end which is exposed to high temperatures. The capillary may further include an adhesive 660 to glue and seal the cap. The heating probe itself may comprise the jacket of the fibre made form, for example acrylate, than a layer of, for example, resin and an outer layer made of, for example, PBT. Additionally, in some examples, the glass capillary 600 may be further bonded 630 to the jacket 620 and/or buffer of the fibre by either fusing or using an adhesive, and/or by shrink tubing.

At the distal end of the bare end fibre where the emitting area is, the space 640 between the fibre and the glass capillary 600 may be filled with air.

This design of the capillary increases the fibre's mechanical stability and its resistance to high temperatures. The structured writing may include the process of ordered writings in periodically repeated cycles, such as at least two cycles are repeated, along the length of the emitting area of an optical fibre. The structured writing is made using a manufacturing process wherein micro-modifications are burnt into the core, and/or cladding and/or buffer of an optical fibre. The micro-modifications may be arranged on one or more sectional planes, wherein the sectional planes lie substantially perpendicular to the optical waveguide axis of the optical fibre. The arrangement of the micro-modifications on the sectional plane by one or more parameters from a group of parameters comprising the symmetric arrangement of the micro-modifications, the density of the micro-modifications on the sectional plane, the size of the micro-modifications, the distance of the micro-modifications from the optical waveguide axis, the distance between the micro-modifications, the alignment of the micro-modifications or other parameters, with the aid of which the position and distribution of the micro-modifications or the size or outer form thereof is described. All these parameters will affect the light transmitted through the fibre and the coupling of the transmitted light out of the fibre thereby providing a diffuse radiation of light from the optical fibre. Each cycle may comprise one or more plane. If a cycle includes more than one plane, different shapes of the cycle may be obtained by vary the parameters, such as each cycle has the shape of, for example a cone or a cylinder, wherein the shape of the cycle may be hollow or filled with micro-modifications. The micro-modifications may have different shapes of the cross sections, for example circular, or ellipsoid. All micro-modifications shaped as ellipsoid may have the same orientation or the orientation may differ between the micro-modifications.

A diffusor may, for example, include two writing cycles, where two cylinders of lesions are generated which are distinct (not overlapping) and in the core of the fibre. The diffusor may be produced with the buffer stripped away in the area of the diffusor.

Another example may be a diffusor with ordered writing, which repeats in a periodic way along the circumference of the core and along the lengths of the diffusor. The diffusor may be produced with the buffer stripped away in the area of the diffusor.

A structured diffusor may also be obtained by creating scattering elements, such as microdots, along the fibre axis. The scattering elements may be arranged close to the boundary of the core of the optical fibre and project into the core for radial decoupling of light. The scattering element may be made either by modified refractive index of the core or as recesses along the core surface by a laser manufacturing method. The recess may in some examples be spherical.

The diffusor may further have the recess filled with a material, such as air, so that a boundary is formed with the corresponding refractive index between the recess and core. The material may, in some examples, include a scattering material having a matrix of scattering particles embedded therein for scattering light. The scattering material may be applied at least area-wise on the optical fibre, such as being coated with the scattering material. The scattering elements may also be applied in the scattering elements.

The scattering elements may be distributed in a peripheral direction and in the longitudinal direction, such as in a spiral, of the diffusor segment of the optical fibre for an even emission of light in a radial direction. The scattering elements may have a variable density, for example the density may increase closer to the distal end of the diffusor. This may be obtained by having the spacing distances decrease towards the distal end.

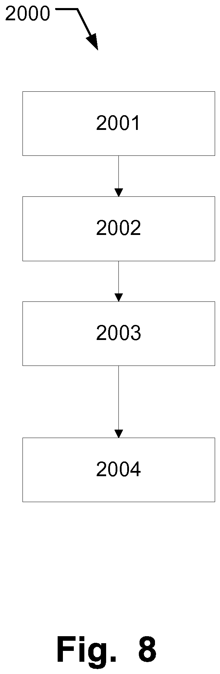

Fig. 8 describes a method 2000 of performing thermotherapy on at least a portion of a tissue site, such as a tumour. The method includes positioning 2001 a sleeve into the tissue to be treated, such as a tumour, wherein the sleeve may include at least one temperature measuring element. Arranging 2002 a heating probe having an energy emitting area inside the sleeve. The heating probe may be an RF probe or MW probe. In some examples the heating probe includes an optical fibre with a light emitting area. The heating probe is connectable to an energy source for heating the tissue through the energy emitting area. The energy source may be a laser unit. Sliding 2003 the sleeve along the heating probe in a distal and/or proximal direction for positioning the energy emitting area in the tissue to be treated. In the examples wherein temperature measuring elements are arranged in the sleeve, sliding 2003 the sleeve along the heating probe in a distal and/or proximal direction may be made to find the optimal distance between at least one temperature measuring element of the sleeve and the energy emitting area. This may be done to determine the size of the treatment lesions which will have a radius about the distance between the temperature measuring element and the energy emitting area. In some examples, the temperature may be measured using a Magnetic Resonance Imaging system to obtain 2D or 3D temperature maps of a treatment area. The treatment area may be used to optimize the size of the treatment lesion.

Controlling 2004 the thermotherapy by monitoring the temperature at the optimal distance from the emitting area and adjusting the power of the energy source.

The optimal distance may be found by checking the temperature at a display while sliding the sleeve along the heating probe. After the optimal distance has been found, the sleeve and heating probe may be locked together to fix the distance by using a hub having a locking member, such as a valve, such as a haemostatic valve.

The present invention has been described above with reference to specific embodiments. However, other embodiments than the above described are equally possible within the scope of the invention. Different method steps than those described above, performing the method by hardware or software, may be provided within the scope of the disclosure. The different features and steps of the invention may be combined in other combinations than those described. The scope of the invention is only limited by the appended patent claims.

The indefinite articles "a" and "an," as used herein in the specification and in the claims, unless clearly indicated to the contrary, should be understood to mean "at least one." The phrase "and/or," as used herein in the specification and in the claims, should be understood to mean "either or both" of the elements so conjoined, i.e., elements that are conjunctively present in some cases and disjunctively present in other cases.

## Claims

1. An apparatus for performing thermotherapy on at least a portion of a tissue site (130, 513), said apparatus comprises:
a heating probe (170, 220, 320, 515, 610) comprising an energy emitting area (180, 270, 370, 514); said heating probe (170, 220, 320, 515, 610) is connectable to an energy source for heating said portion of tissue (130, 513) by said energy emitting area (180, 270, 370, 514);
a sleeve (160, 200, 300, 430, 510) configured to be interstitially inserted in said portion of a tissue (130, 513);
wherein said heating probe (170, 220, 320, 515, 610) is arrangable in said sleeve (160. 200, 300, 430, 510), and said sleeve (160. 200, 300, 430, 510) is configured to be slid along said heating probe (170, 220, 320, 515, 610) in a distal and/or proximal direction for positioning of said energy emitting area (180, 270, 370, 514) in said portion of tissue (130, 513) for controlling said thermotherapy; and said energy emitting area (180, 270, 370, 514) is a light emitting area (180, 270, 370, 514) being an optical fibre which is at least partially a diffuser; said diffuser is a structured writing being micro-modifications ;
**characterized in that** said micro-modifications are burnt into a core and/or
cladding and/or buffer of the optical fibre and said micro-modifications are arranged on one or more sectional planes, wherein said sectional planes lie substantially perpendicular to the optical waveguide axis of the optical fibre.

2. The apparatus of claim 1, wherein said sleeve (160. 200, 300, 430, 510) comprises at least one temperature measuring element (20, 30, 40, 190a, 190b, 260a, 260b, 260c, 360, 530a, 530b, 630c) and said sleeve (160. 200, 300, 430, 510) is configured to be slid along said heating probe (170, 220, 320, 515, 610) in a distal and/or proximal direction for positioning said at least one temperature measuring element (20, 30, 40, 190a, 190b, 260a, 260b, 260c, 360, 530a, 530b, 630c) in relation to said energy emitting area (180, 270, 370, 514).

3. The apparatus of claim 2, wherein said at least one temperature measuring element (20, 30, 40, 190a, 190b, 260a, 260b, 260c, 360, 530a, 530b, 630c) is arranged in a channel of said sleeve (160. 200, 300, 430, 510).

4. The apparatus of any of claims 2 or 3, wherein said at least one temperature measuring element (20, 30, 40, 190a, 190b, 260a, 260b, 260c, 360, 530a, 530b, 630c) is braided or woven into said sleeve (160. 200, 300, 430, 510).

5. The apparatus of any of claims 1 to 4, wherein said sleeve (160. 200, 300, 430, 510) is an introducer catheter.

6. The apparatus of any of claims 2 to 5, wherein said at least one temperature measuring element (20, 30, 40, 190a, 190b, 260a, 260b, 260c, 360, 530a, 530b, 630c) is at least two temperature measuring elements (20, 30, 40, 190a, 190b, 260a, 260b, 260c, 360, 530a, 530b, 630c) arranged at the same transverse plane of said sleeve (160. 200, 300, 430, 510).

7. The apparatus of any of claims 1 to 6, wherein said energy emitting area (180, 270, 370, 514) of said heating probe (170, 220, 320, 515, 610) is covered by a capillary (600).

8. The apparatus of claim 7, wherein fusing, and/or adhering (660), and/or shrink tubing is used to bond (630) said capillary (600) to said heating probe (170, 220, 320, 515, 610).

9. The apparatus of claim 1 to 8, wherein a hub is used to lock said sleeve (160. 200, 300, 430, 510) and said heating probe (170, 220, 320, 515, 610) when right position of said sleeve (160. 200, 300, 430, 510) in relation to said energy emitting area (180, 270, 370, 514) is found.

10. A system for performing thermotherapy on at least a portion of a tissue site (130, 513), said system comprises:
the apparatus for performing thermotherapy on at least a portion of a tissue site (130, 513) according to any of claims 1 to 9;
means for measuring a temperature in said portion of tissue (130, 513); and
a display unit (110) for indicating a measured temperature from said means for measuring a temperature.

11. The system of claim 10, further comprising a control unit (100) for controlling the emitting energy so that said measured temperature is kept at a target temperature between 40 to 60°C.

12. The system of any of claims 10 or 11, wherein said temperature is indicated as a graph of said display (110).

13. The system of claims 10 to 12, wherein said means for measuring a temperature are temperature measuring elements (20, 30, 40, 190a, 190b, 260a, 260b, 260c, 360, 530a, 530b, 630c) arranged in said sleeve (160. 200, 300, 430, 510); or wherein said means for measuring a temperature is a Magnetic Resonance Imaging to obtain a 2D or 3D temperature maps of a treatment area which comprises said portion of tissue (130,513).

## Patentansprüche

1. Eine Vorrichtung zur Durchführung einer Thermotherapie an mindestens einem Gewebeabschnitt (130, 513), wobei die besagte Vorrichtung Folgendes umfasst:
einen Wärmefühler (170, 220, 320, 515, 610) mit einem Energieabgabebereich (180, 270, 370, 514),
wobei besagter Wärmefühler (170, 220, 320, 515, 610) an eine Energiequelle angeschlossen werden kann, um den besagten Gewebeabschnitt (130, 513) über den besagten Energieabgabebereich (180, 270, 370, 514) zu erwärmen,
einen Schlauch (160, 200, 300, 430, 510), der zur interstitiellen Einführung in den besagten Gewebeabschnitt (130, 513) ausgelegt ist,
wobei der besagte Wärmefühler (170, 220, 320, 515, 610) im besagten Schlauch (160, 200, 300, 430, 510) angeordnet werden kann und besagter Schlauch (160, 200, 300, 430, 510) so ausgelegt ist, dass er am besagten Wärmefühler (170, 220, 320, 515, 610) in distaler und/oder proximaler Richtung entlanggleiten kann, um den besagten Energieabgabebereich (180, 270, 370, 514) im besagten Gewebeabschnitt (130, 513) auszurichten und die besagte Thermotherapie zu steuern, und
wobei der besagte Energieabgabebereich (180, 270, 370, 514) ein lichtemittierender Bereich (130, 270, 370, 514) in Form einer Glasfaser ist, die zumindest teilweise einem Diffusor entspricht, wobei besagter Diffusor ein strukturiertes Schreiben in Form von Mikromodifikationen beinhaltet,
**dadurch gekennzeichnet, dass** die besagten Mikromodifikationen in einen Kern und/oder einen Mantel und/oder eine Schutzbeschichtung der Glasfaser gebrannt werden und dass die besagten Mikromodifikationen auf einer oder mehreren Schnittebenen angeordnet sind, wobei die besagten Schnittebenen hauptsächlich senkrecht zur Lichtwellenleiterachse der Glasfaser liegen.

2. Die Vorrichtung gemäß Anspruch 1, wobei der besagte Mantel (160, 200, 300, 430, 510) mindestens ein Temperaturmesselement (20, 30, 40, 190a, 190b, 260a, 260b, 260c, 360, 530a, 530b, 630c) enthält und besagter Mantel (160, 200, 300, 430, 510) so ausgelegt ist, dass er am besagten Wärmefühler (170, 220, 320, 515, 610) in distaler und/oder proximaler Richtung entlanggleiten kann, um das besagte Temperaturmesselement (20, 30, 40, 190a, 190b, 260a, 260b, 260c, 360, 530a, 530b, 630c) in Bezug auf den Energieabgabebereich (180, 270, 370, 514) zu positionieren.

3. Die Vorrichtung gemäß Anspruch 2, wobei das mindestens eine Temperaturmesselement (20, 30, 40, 190a, 190b, 260a, 260b, 260c, 360, 530a, 530b, 630c) in einem Kanal des besagten Mantels (160, 200, 300, 430, 510) angeordnet ist.

4. Die Vorrichtung gemäß einem der Ansprüche 2 oder 3, wobei das besagte mindestens eine Temperaturmesselement (20, 30, 40, 190a, 190b, 260a, 260b, 260c, 360, 530a, 530b, 630c) in den besagten Mantel (160, 200, 300, 430, 510) eingeflochten oder eingewebt ist.

5. Die Vorrichtung gemäß einem der Ansprüche 1 bis 4, wobei der besagte Mantel (160, 200, 300,430, 510) ein Einführkatheter ist.

6. Die Vorrichtung gemäß einem der Ansprüche 2 bis 5, wobei das besagte mindestens eine Temperaturmesselement (20, 30, 40, 190a, 190b, 260a, 260b, 260c, 360, 530a, 530b, 630c) aus mindestens zwei Temperaturmesselementen (20, 30, 40, 190a, 190b, 260a, 260b, 260c, 360, 530a, 530b, 630c) besteht, die auf der gleichen Querebene des besagten Mantels (160, 200, 300, 430, 510) angeordnet sind.

7. Die Vorrichtung gemäß einem der Ansprüche 1 bis 6, wobei der besagte Energieabgabebereich (180, 270, 370, 514) des besagten Wärmefühlers (170, 220, 320, 515, 610) von einer Kapillare (600) bedeckt ist.

8. Die Vorrichtung gemäß Anspruch 7, wobei Schweißen und/oder Haften (660) und/oder Wärmeschrumpfen zum Anbinden besagter Kapillare (600) an besagten Wärmefühler (170, 220, 320, 515, 610) verwendet wird.

9. Die Vorrichtung gemäß Anspruch 1 bis 8, wobei ein Hub verwendet wird, um den besagten Mantel (160, 200, 300, 430, 510) und besagten Wärmefühler (170, 220, 320, 515, 610) zu verriegeln, wenn die richtige Position des besagten Mantels (160, 200, 300, 430,510) in Bezug auf den Energieabgabebereich (180, 270, 370, 514) gefunden ist.

10. Ein System zur Durchführung einer Thermotherapie an mindestens einem Gewebeabschnitt (130, 513), wobei das besagte System Folgendes umfasst:
die Vorrichtung zur Durchführung einer Thermotherapie an mindestens einem Gewebeabschnitt (130, 513) gemäß einem der Ansprüche 1 bis 9,
ein Gerät zur Messung der Temperatur im besagten Gewebeabschnitt (130, 513) und
eine Anzeigeeinheit (110) zur Anzeige der durch besagtes Temperaturmessgerät gemessenen Temperatur.

11. Das System gemäß Anspruch 10, das weiterhin eine Kontrolleinheit (100) zur Kontrolle der ausgegebenen Energie umfasst, so dass die besagte gemessene Temperatur auf einer Solltemperatur zwischen 40 und 60°C gehalten wird.

12. Das System gemäß einem der Ansprüche 10 oder 11, wobei besagte Temperatur in Form einer Kurve auf der besagten Anzeige wiedergegeben wird.

13. Das System gemäß einem der Ansprüche 10 bis 12, wobei das besagte Gerät zur Messung der Temperatur Temperaturmesselementen (20, 30, 40, 190a, 190b, 260a, 260b, 260c, 360, 530a, 530b, 630c) entspricht, die im besagten Mantel (160, 200, 300, 430, 510) abgeordnet sind, oder wobei das besagte Gerät zur Messung der Temperatur einer Kernspintomografie zur Erstellung von 2D- oder 3D-Temperaturkarten des Behandlungsbereichs einschließlich des besagten Gewebeabschnitts (130, 513) entspricht.

## Revendications

1. Appareil destiné à réaliser une thermothérapie sur au moins une partie d'un site de tissu (130, 513), ledit appareil comprend :
une sonde de chauffage (170, 220, 320, 515, 610) comprenant une zone d'émission d'énergie (180, 270, 370, 514) ; ladite sonde de chauffage (170, 220, 320, 515, 610) peut être connectée à une source d'énergie pour chauffer ladite partie de tissu (130, 513) au moyen de ladite zone d'émission d'énergie (180, 270, 370, 514) ;
un manchon (160, 200, 300, 430, 510) configuré pour être inséré de manière interstitielle dans ladite partie d'un tissu (130, 513) ;
dans lequel ladite sonde de chauffage (170, 220, 320, 515, 610) peut être agencée dans ledit manchon (160, 200, 300, 430, 510), et ledit manchon (160, 200, 300, 430, 510) est configuré pour être glissé le long de ladite sonde de chauffage (170, 220, 320, 515, 610) dans une direction distale et/ou proximale pour le positionnement de ladite zone d'émission d'énergie (180, 270, 370, 514) dans ladite partie de tissu (130, 513) afin de commander ladite thermothérapie ; et
ladite zone d'émission d'énergie (180, 270, 370, 514) est une zone d'émission de lumière (180, 270, 370, 514) qui est une fibre optique laquelle est au moins en partie un diffuseur ; ledit diffuseur est une inscription structurée qui est des micro-modifications ;
**caractérisé en ce que** lesdites micro-modifications sont brûlées dans un cœur et/ou une gaine et/ou une couche protectrice de la fibre optique et lesdites micro-modifications sont agencées sur un ou plusieurs plans de coupe, où lesdits plans de coupe se situent sensiblement perpendiculairement à l'axe de guide d'onde optique de la fibre optique.

2. Appareil selon la revendication 1, dans lequel ledit manchon (160, 200, 300, 430, 510) comprend au moins un élément de mesure de température (20, 30, 40, 190a, 190b, 260a, 260b, 260c, 360, 530a, 530b, 630c) et ledit manchon (160, 200, 300, 430, 510) est configuré pour être glissé le long de ladite sonde de chauffage (170, 220, 320, 515, 610) dans une direction distale et/ou proximale pour positionner ledit au moins un élément de mesure de température (20, 30, 40, 190a, 190b, 260a, 260b, 260c, 360, 530a, 530b, 630c) par rapport à ladite zone d'émission d'énergie (180, 270, 370, 514).

3. Appareil selon la revendication 2, dans lequel ledit au moins un élément de mesure de température (20, 30, 40, 190a, 190b, 260a, 260b, 260c, 360, 530a, 530b, 630c) est agencé dans un canal dudit manchon (160, 200, 300, 430, 510).

4. Appareil selon l'une quelconque des revendications 2 et 3, dans lequel ledit au moins un élément de mesure de température (20, 30, 40, 190a, 190b, 260a, 260b, 260c, 360, 530a, 530b, 630c) est tressé ou tissé dans ledit manchon (160, 200, 300, 430, 510).

5. Appareil selon l'une quelconque des revendications 1 à 4, dans lequel ledit manchon (160, 200, 300, 430, 510) est un cathéter d'introduction.

6. Appareil selon l'une quelconque des revendications 2 à 5, dans lequel ledit au moins un élément de mesure de température (20, 30, 40, 190a, 190b, 260a, 260b, 260c, 360, 530a, 530b, 630c) est au moins deux éléments de mesure de température (20, 30, 40, 190a, 190b, 260a, 260b, 260c, 360, 530a, 530b, 630c) agencés au niveau du même plan transversal dudit manchon (160, 200, 300, 430, 510).

7. Appareil selon l'une quelconque des revendications 1 à 6, dans lequel ladite zone d'émission d'énergie (180, 270, 370, 514) de ladite sonde de chauffage (170, 220, 320, 515, 610) est recouverte d'un capillaire (600).

8. Appareil selon la revendication 7, dans lequel une fusion, et/ou un collage (660), et/ou un tube thermorétrécissable est utilisé pour coller (630) ledit capillaire (600) sur ladite sonde de chauffage (170, 220, 320, 515, 610).

9. Appareil selon les revendications 1 à 8, dans lequel un moyeu est utilisé pour bloquer ledit manchon (160, 200, 300, 430, 510) et ladite sonde de chauffage (170, 220, 320, 515, 610) lorsqu'une bonne position dudit manchon (160, 200, 300, 430, 510) par rapport à ladite zone d'émission d'énergie (180, 270, 370, 514) est trouvée.

10. Système destiné à réaliser une thermothérapie sur au moins une partie d'un site de tissu (130, 513), ledit système comprend :
l'appareil destiné à réaliser une thermothérapie sur au moins une partie d'un site de tissu (130, 513) selon l'une quelconque des revendications 1 à 9 ;
un moyen destiné à mesurer une température dans ladite partie de tissu (130, 513) ; et
une unité d'affichage (110) destinée à indiquer une température mesurée à partir dudit moyen destiné à mesurer une température.

11. Système selon la revendication 10, comprenant en outre une unité de commande (100) destinée à commander l'émission d'énergie de sorte que ladite température mesurée est maintenue à une température cible comprise entre 40 et 60 °C.

12. Système selon l'une quelconque des revendications 10 et 11, dans lequel ladite température est indiquée sous la forme d'un graphique dudit affichage (110).

13. Système selon les revendications 10 à 12, dans lequel lesdits moyens destinés à mesurer une température sont des éléments de mesure de température (20, 30, 40, 190a, 190b, 260a, 260b, 260c, 360, 530a, 530b, 630c) agencés dans ledit manchon (160, 200, 300, 430, 510); ou dans lequel ledit moyen destiné à mesurer une température est une imagerie par résonance magnétique pour obtenir des cartographies de température 2D ou 3D d'une zone de traitement laquelle comprend ladite partie de tissu (130, 513).
